# EUROPEAN PATENT SPECIFICATION

(11) **EP 1 745 765 B1**
(45) Date of publication and mention of the grant of the patent: **06.04.2011**
(21) Application number: 06076851.2
(22) Date of filing: 05.09.2002
(51) Int. Cl.: A61F 5/00, A61B 17/00

(54) **Stoma opening forming apparatus with connection device**
Gerät zur Bildung einer Stomaöffnung mit Verbindungsvorrichtung
Appareil permettant de former un orifice de stomie et doté d'un appareil de connexion

(30) Priority: 05.09.2001 US 316987 P
(43) Date of publication of application: 24.01.2007
(62) Divisional of application: 02797720.6
(73) Proprietor: Potencia Medical AG, 6341 Baar (CH)
(72) Inventor: Forsell, Peter, 6300 Zug (CH)
(74) Representative: Mercer, Christopher Paul

(56) References cited:
- EP-A- 1 036 545
- WO-A-01/49245
- FR-A- 2 797 181
- FR-A- 2 802 406
- US-A- 4 558 699

## Description

The present invention relates to an apparatus for forming a stoma opening in the stomach or oesophagus of a patient. The apparatus comprises a constriction device adapted to be applied on the patient's stomach or oesophagus and including an elongate adjustable constriction member adapted to extend in a loop around and constrict the stomach or oesophagus to form the stoma opening therein, the constriction member having first and second end portions. The apparatus further comprises a connection device for releasably connecting the first and second end portions of the constriction member to each other, and an adjustment device that adjusts the longitudinal extension of the constriction member in said loop to change the size of the stoma opening

This kind of apparatus in the form of a gastric banding device, in which a band encircles a portion of a patient's stomach to restrict the food intake of the patient, have been used in surgery for morbid obesity to form a small gastric pouch above the band and a reduced stoma opening in the stomach. Although such a band is applied around the stomach to obtain an optimal stoma opening during surgery, some prior gastric banding devices are provided with an adjustment device enabling a minor post-operation adjustment of the size of the stoma opening.

The kind of apparatus presented initially has also been used for treating heartburn and reflux disease due to hiatal hernia, *i.e.* a portion of the stomach immediately below the gastric fundus slides upwardly through the esophageal hiatus. In consequence, stomach acids and foods are regurgitated into the oesophagus. In the late 1970s a prior art prosthesis called Angelchik, according to U.S. Patent No. 3.875 928, was used to operatively treat heartburn and reflux disease. However, the Angelchik prosthesis had a major disadvantage in that it was not possible to adjust the size of the restriction opening after the operation. A further disadvantage was that the prosthesis did not satisfactorily protect the oesophagus and the surrounding area against injuries due to poor shape of the prosthesis. Therefore, operations using the Angelchik prosthesis are no longer practised.

An operation technique, semi-fundoduplicatio, is currently in use for treating heartburn and reflux disease. A most common operation is Nissen semi-fundoduplicatio, in which one takes the fundus of the stomach and makes a three quarter of a turn around the oesophagus and sutures between the stomach and oesophagus. Although this operation works fairly well it has three main disadvantages. Firstly, most patients treated in accordance to semi-fundoduplicatio lose their ability to belch. Secondly, many of these patients get dysphagia, *i.e.* difficulties to swallow after the operation. Thirdly, it is not possible to adjust the stoma opening in the oesophagus or stomach in any way after the operation. Characteristic for these patients is the variation of their problems over the day. For example, many patients have difficulties during the night when they lie down because of stomach acid leaking up into the oesophagus.

FR 2 797 181 discloses a gastric band according to the pre-amble of claim 1.

The prime object of the present invention is to provide a new adjustable apparatus designed to form an adjustable stoma opening in the stomach or oesophagus of a patient, wherein the new apparatus is suited for treating obese patients as well as patients suffering from heartburn and reflux disease.

Another object of the present invention is to provide a new convenient apparatus for forming a stoma opening, which is easy to apply around the stomach or oesophagus and easy to remove from the stomach or oesophagus.

These objects are obtained by an apparatus of the kind presented initially characterised in that the connection device includes a female part and a male part fitting into the female part to lock them together.

The male part includes a shank and a head on the shank and the female part includes two jaws defining a bore for receiving the head of the male part and a passage narrower than and extending from the bore for receiving the shank of the male part, whereby the male part can be connected to the female part by laterally displacing the head and shank of the male part into the bore and passage, respectively. The jaws are formed with indentations in the bore adjacent the passage fitting the head of the male part, such that the head can be displaced laterally relative to the bore into the indentations to lock the male and female parts to each other.

The constriction member may be non-inflatable and the adjustment device may include a motor for adjusting the non-inflatable constriction member. Alternatively, the constriction member may be a hydraulic constriction member, typically with an inflatable cavity, and the adjustment device may include a pump hydraulically connected to the hydraulic constriction member.

Generally, the adjustment device comprises a powered adjustment device, for example including a motor, preferably an electric motor. The apparatus may comprise an implantable energy-transforming device adapted to transform wireless energy emitted from outside the patient's body into an energy form suited for powering the adjustment device. Such an energy form may be electric energy for powering an electric motor of the adjustment device.

To conveniently adjust the size of the stoma opening the apparatus may comprise a wireless remote control for controlling the adjustment device from outside the patient's body to adjust the constriction device to change the size of the stoma opening.

In the enclosed drawings:
Figure 1 is a view of the apparatus of the invention with a connection device,
Figure 2 is a schematic view of the connection device in an unlocked state,
Figure 3 is a schematic view of the connection device in a pre-locked state,
Figure 4 is a schematic view of the connection device in a final locked state, and
Figure 5 illustrates the apparatus of the invention implanted in an obese patient.

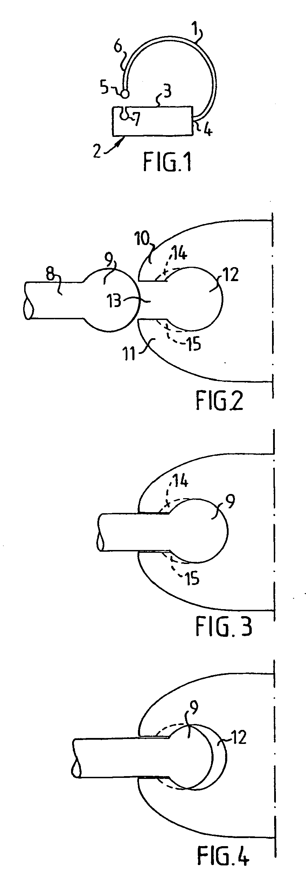

Figure 1 shows the apparatus of the present invention including a constriction device having an elongated constriction member 1 to be formed into a closed loop around a patient's stomach or oesophagus to form a stoma opening therein. An adjustment device 2 includes an elongate housing 3 connected to an end portion 4 of the constriction member 1. There is a connection device in the form of a male part 5 on another end portion 6 of the constriction member 1 and a female part 7 formed on the housing 3. (Alternatively, the male part 5 may be provided on the housing 3 and the female part 7 may be formed on the end portion 6.)

With reference to Figures 2 - 4, the male part 5 has a shank 8 and a spherical head 9 on the shank 8 and the female part 7 has two jaws 10,11 defining a circular bore 12 for receiving the head 9 and a passage 13 narrower than and extending from the bore 12 for receiving the shank 8. As appears from Figure 3 the male part 5 can be pre-locked to the female part 7 by laterally displacing the head 9 and shank 8 into the bore 12 and passage 13, respectively. The jaws 10,11 are formed with spherical segment indentations 14,15 complementary to the spherical head 9. The indentations 14,15 are located in the bore 12 adjacent the passage 13. As appears from Figure 4 the male part 5 can be finally locked to the female part 7 by displacing the head 9 laterally relative to the bore 12 into the indentations 14,15. The pressure that the stomach or oesophagus constantly exerts on the constriction member when the apparatus is implanted ensures that the connection device 5,7 is kept in a locked state.

In case the apparatus should be removed from the patient, the surgeon may conveniently use laparoscopic surgery to introduce a suitable tool into the patient's abdomen to release the head 9 from the jaws 11,11 and displace the head 9 out of the bore 12.

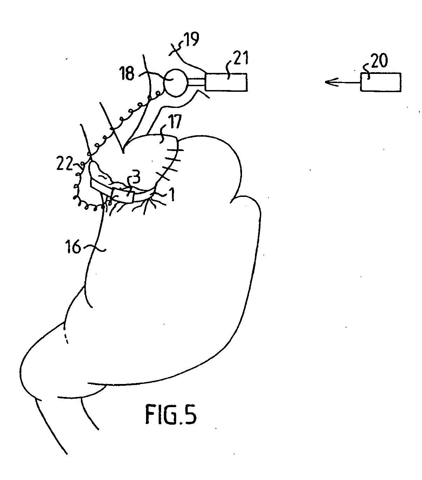

Figure 5 illustrates the constriction device the embodiment shown in Figures 1-4 applied on the stomach 16 of an obese patient. The elongate constriction member 1 and housing 3 of the constriction device extend in a loop around and constricts the stomach 16 to form an upper pouch 17 of the stomach 16 and a restricted stoma opening in the stomach 17. A rechargeable electric power supply 18 is implanted in the patient and fixed to the breastbone 19 (the sternum). An external remote control 20 controls the adjustment device 2 and transmits signals that are received by a combined control and energy transforming unit 21 subcutaneously implanted in the patient. The unit 21 is electrically connected to the electric power supply 18 and transforms the energy of the signals into an electric current that is used for charging the electric power supply 18. For example, the signals may include electromagnetic waves and the unit 21 may include an electric p-n junction element that transforms the wireless energy into an electric current. A resilient insulated electric wire 22 connects the power supply 18 and an electric motor of the adjustment device 2 contained in the housing 3. The electric wire 22 extends helically between the power supply 18 and housing 3, in order to permit the electric wire 22 to be temporarily extended when dynamic movements of the stomach 16 and oesophagus occur, so that the risk of breaking the electric wire 22 is eliminated.

## Claims

1. An apparatus for forming a stoma opening in the stomach (16) or oesophagus of a patient, the apparatus comprising a constriction device (1,3) adapted to be applied on the patient's stomach or oesophagus, the constriction device including an elongate adjustable constriction member (1) adapted to extend in a loop around and constrict the stomach or oesophagus to form the stoma opening therein, the constriction member having first and second end portions (4,6), a connection device (5,7) for releasably connecting the first and second end portions of the constriction member to each other, and an adjustment device (2) that adjusts the longitudinal extension of the constriction member (1) in said loop to change the size of the stoma opening, wherein the connection device includes a female part (7) and a male part (5) fitting into the female part to lock them together,
**characterised in that**: the male part (5) comprises a shank (8) and a head (9) on the shank and the female part (7) comprises two jaws (10,11) defining a bore (12) for receiving the head of the male part and a passage (13) narrower than and extending from the bore for receiving the shank of the male part, whereby the male part can be connected to the female part by laterally displacing the head and shank of the male part into the bore and passage, respectively, wherein the jaws (10,11) are formed with indentations (14,15) in the bore (12) adjacent the passage (13) fitting the head (9) of the male part (5), such that the head can be displaced laterally relative to the bore into the indentations to lock the male and female parts to each other.

2. An apparatus according to claim 1, wherein the constriction member (1) is non-inflatable.

3. An apparatus according to claim 2, wherein the adjustment device (2) comprises a motor.

4. An apparatus according to claim 1, wherein the constriction member comprises a hydraulic constriction member and the adjustment device (2) comprises a pump hydraulically connected to the hydraulic constriction member.

5. An apparatus according to any of claims 1-4, wherein the adjustment device (2) comprises a powered adjustment device and further comprising an implantable energy transforming device (21) adapted to transform wireless energy emitted from outside the patients body into an energy form suited for powering the adjustment device.

6. An apparatus according to any of claims 1-5, further comprising a wireless remote control (20) for controlling the adjustment device (2) to adjust the constriction device (1,3) to change the size of the stoma opening.

## Patentansprüche

1. Vorrichtung zum Bilden einer Stomaöffnung in dem Magen (16) oder dem Ösophagus eines Patienten, wobei die Vorrichtung eine Einengungseinrichtung (1, 3), welche eingerichtet ist, um auf den Magen oder den Ösophagus des Patienten angewendet zu werden, umfasst, wobei die Einengungseinrichtung ein längliches, einstellbares Einengungselement (1), welches eingerichtet ist, um sich in einer Schlaufe um den Magen oder den Ösophagus zu erstrecken und diesen einzuengen, um die Stomaöffnung darin zu bilden, wobei das Einengungselement einen ersten und einen zweiten Endbereich (4, 6) aufweist, eine Verbindungseinrichtung (5, 7) zum lösbaren miteinander Verbinden des ersten und des zweiten Endbereichs des Einengungselements und eine Einstelleinrichtung (2), welche die Längserstreckung des Einengungselements (1) in der Schlaufe einstellt, um die Größe der Stomaöffnung zu verändern, aufweist, wobei die Verbindungseinrichtung ein Mutterteil (7) und ein Vaterteil (5), welches in das Mutterteil passt, um sie aneinander festzumachen, aufweist, **dadurch gekennzeichnet, dass**:
das Vaterteil (5) einen Schaft (8) und einen Kopf (9) auf dem Schaft umfasst, und dass das Mutterteil (7) zwei Klauen (10, 11), welche zum Aufnehmen des Kopfes des Vaterteils eine Bohrung (12) und zum Aufnehmen des Schaftes des Vaterteils einen Durchgang (13), welcher enger als die Bohrung ist und sich davon erstreckt, umfasst, wodurch das Vaterteil durch ein laterales Verschieben des Kopfes und des Schaftes des Vaterteils in die Bohrung bzw. den Durchgang mit dem Mutterteil verbunden werden kann,
wobei die Klauen (10, 11) mit Vertiefungen (14, 15) in der Bohrung (12) neben dem Durchgang (13), welche für den Kopf (9) des Vaterteils (5) passen, ausgestaltet sind, so dass der Kopf lateral relativ zu der Bohrung in die Vertiefungen verschoben werden kann, um das Vater- und das Mutterteil aneinander festzumachen.

2. Vorrichtung gemäß Anspruch 1, wobei das Einengungselement (1) nicht aufblasbar ist.

3. Vorrichtung gemäß Anspruch 2, wobei die Einstelleinrichtung (2) einen Motor umfasst.

4. Vorrichtung gemäß Anspruch 1, wobei das Einengungselement ein hydraulisches Einengungselement umfasst und die Einstelleinrichtung (2) eine Pumpe, welche hydraulisch mit dem hydraulischen Einengungselement verbunden ist, umfasst.

5. Vorrichtung gemäß einem der Ansprüche 1-4, wobei die Einstelleinrichtung (2) eine angetriebene Einstelleinrichtung umfasst und weiter eine implantierbare energieumwandelnde Einrichtung (21), welche eingerichtet ist, um drahtlose Energie, welche von außerhalb des Patientenkörpers ausgesandt wird, in eine Energieform, welche zum Antreiben der Einstellungseinrichtung geeignet ist, umzuwandeln, umfasst.

6. Vorrichtung gemäß einem der Ansprüche 1-5, welche weiter eine drahtlose Fernsteuerung (20) zum Steuern der Einstelleinrichtung (2) zum Einstellen der Einengungseinrichtung (1, 3) für ein Ändern der Größe der Stomaöffnung umfasst.

## Revendications

1. Appareil pour former un orifice de stomie dans l'estomac (16) ou dans l'oesophage d'un patient, l'appareil comprenant un dispositif de constriction (1, 3) apte à être appliqué à l'estomac ou à l'oesophage du patient, le dispositif de constriction comportant un élément de constriction ajustable oblong (1) apte à s'étendre dans une boucle autour et à contraindre l'estomac ou l'oesophage pour former l'orifice de stomie dans celui-ci, l'élément de constriction ayant des première et seconde portions d'extrémité (4, 6), un dispositif de connection (5, 7) pour relier relâchablement les première et seconde portions d'extrémité de l'élément de constriction l'une à l'autre, et un dispositif d'ajustement (2) qui ajuste l'extension longitudinale de l'élément de constriction (1) dans ladite boucle pour changer la taille de l'orifice de stomie, où le dispositif de connection comporte une partie femelle (7) et une partie mâle (5) s'ajustant dans la partie femelle pour les verrouiller ensemble, **caractérisé en ce que** la partie mâle (5) comprend un fût (8) et une tête (9) sur le fût, et la partie femelle (7) comprend deux mâchoires (10, 11) définissant un perçage (12) pour recevoir la tête de la partie mâle, et un passage (13) plus étroit que et s'étendant du perçage pour recevoir le fût de la partie mâle, moyennant quoi la partie mâle peut être reliée à la partie femelle en déplaçant latéralement la tête et le fût de la partie mâle dans le perçage et le passage, respectivement, où les mâchoires (10, 11) présentent des indentations (14, 15) dans le perçage (12) adjacent au passage (13) ajustant la tête (9) de la partie mâle (5) de sorte que la tête peut être déplacée latéralement relativement au perçage dans les indentations pour verrouiller les parties mâle et femelle l'une à l'autre.

2. Appareil selon la revendication 1, dans lequel l'élément de constriction (1) n'est pas gonflable.

3. Appareil selon la revendication 2, dans lequel le dispositif d'ajustement (2) comprend un moteur.

4. Appareil selon la revendication 1, dans lequel l'élément de constriction comprend un élément de constriction hydraulique, et le dispositif d'ajustement (2) comprend une pompe reliée hydrauliquement à l'élément de constriction hydraulique.

5. Appareil selon l'une quelconque des revendications 1 à 4, dans lequel le dispositif d'ajustement (2) comprend un dispositif d'ajustement motorisé, et comprenant en outre un dispositif de transformation d'énergie implantable (21) apte à transformer l'énergie sans fil émise depuis l'extérieur du corps du patient en une forme d'énergie qui convient pour alimenter le dispositif d'ajustement.

6. Appareil selon l'une quelconque des revendications 1 à 5, comprenant en outre une commande à distance sans fil (20) pour commander le dispositif d'ajustement (2) pour ajuster le dispositif de constriction (1, 3) afin de changer la taille de l'orifice de stomie.
